# EUROPEAN PATENT APPLICATION

(11) **EP 4 305 962 A1**
(43) Date of publication of application: **17.01.2024**
(21) Application number: 22766778.9
(22) Date of filing: 18.02.2022
(51) Int. Cl.: A01N 43/16, A01P 1/00, A61K 8/60, A61K 31/7028, A61K 31/7032, A61L 2/16, A61L 2/18, A61L 2/23, A61P 31/12, A61P 31/14, A61P 31/16, A61P 31/22, A61Q 5/02, A61Q 19/00, A61Q 19/10, C11D 1/68

(54) **ANTIVIRAL AGENT**

(30) Priority: 08.03.2021 JP 2021036197; 21.09.2021 JP 2021153308
(71) Applicant: TOYOBO CO., LTD., Osaka-shi, Osaka 530-0001 (JP)
(72) Inventor: KAWAHARA, Akio, Tsuruga-shi, Fukui 914-8550 (JP); YAMAMOTO, Shuhei, Osaka-shi, Osaka 530-8230 (JP)
(74) Representative: Vossius & Partner Patentanwälte Rechtsanwälte mbB
(86) International application number: PCT/JP2022/006587
(87) International publication number: WO 2022/190815

(57) **Abstract**

Provided is an antiviral agent that is suitable not only for application in articles but also for application in living organisms, and that is safer for living organisms. The antiviral agent contains mannosylerythritol lipids (MELs) as an active ingredient, preferably in an amount of 0.000001 to 100 wt%, and is applicable to living organisms and/or articles.

## Description

### Technical Field

The present invention relates to antiviral agents. More specifically, the present invention relates to antiviral agents that are suitable not only for application in articles but also for application in living organisms, and that are safer for living organisms.

### Background Art

Viruses are classified as non-cellular organisms, which are distinct from organisms, and propagate by infecting the cells of hosts such as mammals and birds. Viruses cause infectious diseases, such as foot-and-mouth disease and avian influenza, and are becoming social problems. With the improvement of the living environment and changes in terms of hygienic awareness, there has been demand for the development of substances excellent in antiviral action and virus-inactivating action, such as those capable of inactivating viruses existing in the environment.

Various methods for inactivating viruses in the environment to prevent viral infections have been studied. Examples include physical treatments such as heat treatment and UV treatment, and treatments with chemicals such as chlorine bleach and peroxides. These treatments are difficult to use safely and in a wide range of situations because of the potential for damage to living organisms and articles. Thus, there is a strong demand for antiviral agents or virus-inactivating agents that are suitable not only for application in articles but also for application in living organisms and that are safer for living organisms, especially in pandemic situations.

Mannosylerythritol lipids (MELs) are natural surfactants produced by yeast, and their various physiological actions (NPL 1) and antibacterial actions (PTL 1) are reported. Research also reports that application of MELs in topical medication or cosmetic products improves rough skin (PTL 2). Thus, MELs are considered to be a material that has various physiological activities and is also highly safe for living organisms. However, the antiviral or virus-inactivating actions of MELs have not previously been examined.

### Citation List

### Patent Literature

PTL 1: JPS57-145896A
PTL 2: WO2007/060956A

### Non-patent Literature

NPL 1: Journal of Bioscience and Bioengineering, 94, 187 (2002)
NPL 2: Biologicals, 25, 3, pp. 289-297 (1997)

### Summary of Invention

### Technical Problem

An object of the present invention is to provide an antiviral agent that is suitable not only for application in articles but also for application in living organisms, and that is safer for living organisms.

### Solution to Problem

The present inventors found that MELs, a type of biosurfactants, have an excellent antiviral effect; and completed the present invention.

Examples of specific embodiments of the present invention specifically include the following.

### Item 1.

An antiviral agent comprising a mannosylerythritol lipid (MEL) as an active ingredient.

### Item 2.

The antiviral agent according to Item 1, wherein the content of the mannosylerythritol lipid (MEL) is 0.000001 to 100 wt%.

### Item 3.

The antiviral agent according to Item 2, wherein the content of the mannosylerythritol lipid (MEL) is 0.000001 to 80 wt%.

### Item 4.

The antiviral agent according to Item 2, wherein the content of the mannosylerythritol lipid (MEL) is 0.0001 to 10 wt%.

### Item 5.

The antiviral agent according to any one of Items 1 to 4, wherein the mannosylerythritol lipid (MEL) is any one selected from the group consisting of MEL-A, MEL-B, MEL-C, and MEL-D. Item 6.

The antiviral agent according to any one of Items 1 to 5, wherein the mannosylerythritol lipid (MEL) has a structure of formula (2): 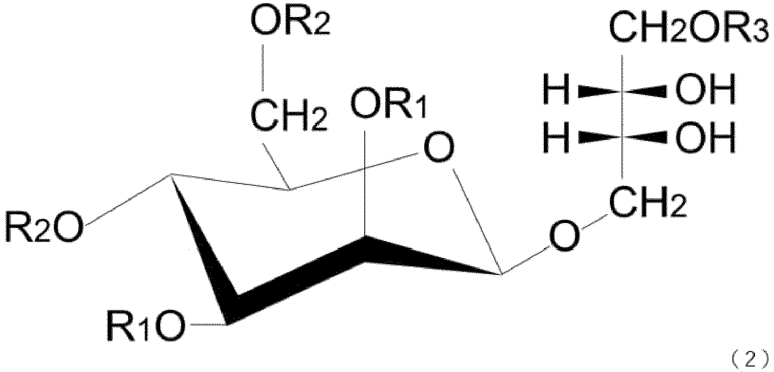 wherein R₁s are an aliphatic acyl group having 4 to 24 carbon atoms and may be identical to or different from each other, R₂s are a hydrogen atom or an acetyl group and may be identical to or different from each other, and R₃ is a hydrogen atom or an aliphatic acyl group having 2 to 24 carbon atoms. Item 7.

The antiviral agent according to any one of Items 1 to 5, wherein the mannosylerythritol lipid (MEL) has a structure of formula (3): 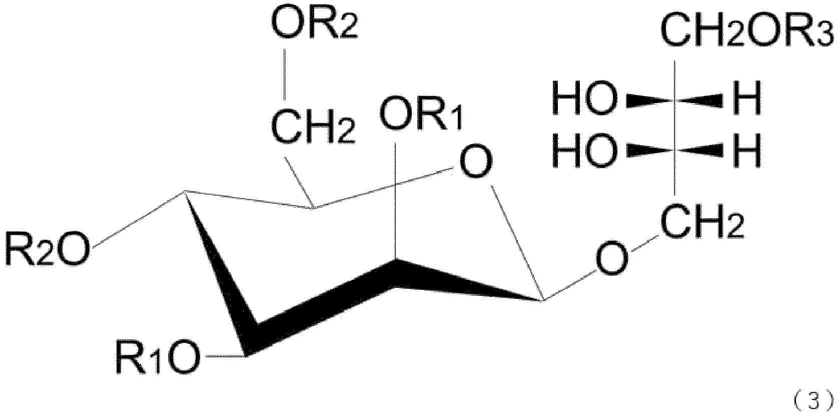 wherein R₁s are an aliphatic acyl group having 4 to 24 carbon atoms and may be identical to or different from each other, R₂s are a hydrogen atom or an acetyl group and may be identical to or different from each other, and R₃ is a hydrogen atom or an aliphatic acyl group having 2 to 24 carbon atoms.

### Item 8.

The antiviral agent according to any one of Items 1 to 5, wherein the mannosylerythritol lipid (MEL) is MEL-B. Item 9.

The antiviral agent according to Item 8, wherein the MEL-B has a structure of formula (4): 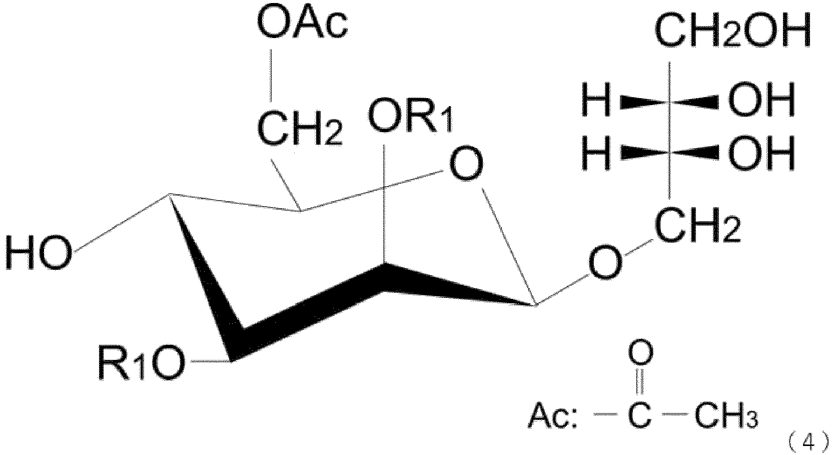 wherein R₁s are a saturated or unsaturated, linear or branched aliphatic acyl group having 2 to 20 carbon atoms and may be identical to or different from each other.

### Item 10.

The antiviral agent according to Item 8, wherein the MEL-B has a structure of formula (5): 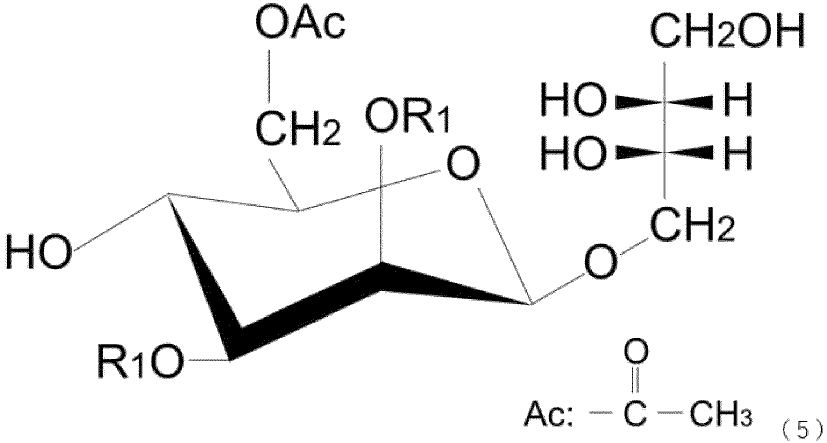 wherein R₁s are a saturated or unsaturated, linear or branched aliphatic acyl group having 2 to 20 carbon atoms and may be identical to or different from each other.

### Item 11.

The antiviral agent according to any one of Items 1 to 10, wherein a target virus is an enveloped virus.

### Item 12.

The antiviral agent according to Item 11, wherein the target virus is an influenza virus.

### Item 13.

The antiviral agent according to Item 11, wherein the target virus is a human coronavirus.

### Item 14.

The antiviral agent according to any one of Items 1 to 13, which is for use in a living organism.

### Item 15.

The antiviral agent according to any one of Items 1 to 13, which is for use in an article.

### Item 16.

A cosmetic product comprising the antiviral agent of any one of Items 1 to 14.

### Item 17.

A disinfectant comprising the antiviral agent of any one of Items 1 to 15.

### Item 18.

A cleaner comprising the antiviral agent of any one of Items 1 to 15.

### Advantageous Effects of Invention

The use of the composition containing an MEL according to the present invention provides an antiviral agent that is suitable not only for application in articles but also for application in living organisms and that is safer for living organisms.

### Brief Description of Drawings

Fig. 1 is a graph showing the results of examining a virus-inactivating effect on a human coronavirus in Example 1.
Fig. 2 is a graph showing the results of examining a virus-inactivating effect on an influenza virus in Example 1.

### Description of Embodiments

In the present invention, the term "antiviral agent" refers to a chemical agent effective in treating or preventing viral infections by limiting the proliferation of a virus or by eliminating the infectivity of a virus attached to a living organism or an article. The destruction of the external tissues of a virus is considered to lead the virus to lose its functionality of invading the cells of organisms and proliferating and to render the virus inactive.

"Biosurfactant" is a generic term for substances with surface-activating and emulsifying capabilities produced by living organisms. Due to their excellent surface-activating capability and high biodegradability as well as various physiological actions, biosurfactants may exhibit behaviors and functions different from those of synthetic surfactants. Currently, biosurfactants are classified into five categories: glycolipids, acyl-peptides, phospholipids, fatty acids, and polymeric compounds. Glycolipid biosurfactants are composed of carbohydrate and fatty acid moieties, and preferable examples include mannosylerythritol lipids (MELs). The MEL for use in the present invention can be of any type, and examples include MEL-A, MEL-B, MEL-C, and MEL-D, with MEL-B being particularly preferable.

The structure of MELs is shown in formula (1). In formula (1), substituents R₁ may be identical to or different from each other and are an aliphatic acyl group having 4 to 24 carbon atoms. MELs are classified into four types, MEL-A, MEL-B, MEL-C, and MEL-D, based on whether an acetyl group is present at positions 4 and 6 of mannose.

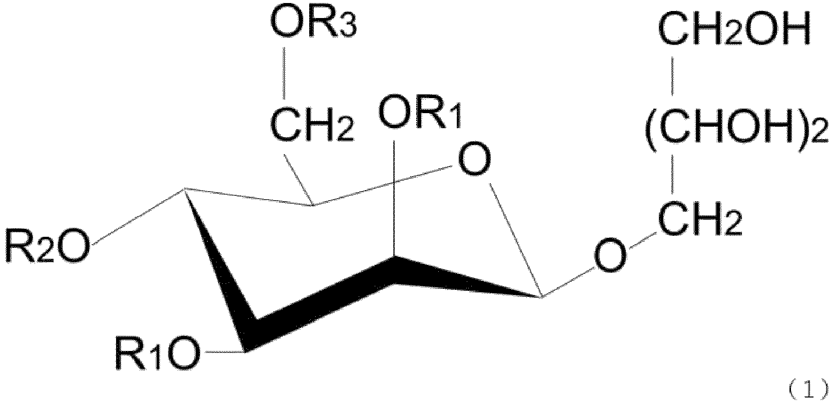

Specifically, in MEL-A, substituents R₂ and R₃ in formula (1) are both an acetyl group. In MEL-B, substituent R₂ is an acetyl group and substituent R₃ is a hydrogen atom in formula (1). In MEL-C, substituent R₂ is a hydrogen atom, and substituent R₃ is an acetyl group in formula (1). In MEL-D, both substituents R₂ and R₃ are a hydrogen atom in formula (1) .

The number of carbon atoms of substituents R₁ in MEL-A to MEL-D varies depending on the number of carbon atoms of fatty acids constituting triglycerides, which are fats and oils contained in an MEL-producing medium, and the degree of assimilation of fatty acids by a used MEL-producing yeast. Additionally, when the triglycerides have unsaturated fatty acid residues, unsaturated fatty acid residues may be contained as substituent R₁ unless the MEL-producing yeast assimilates the double bond portion of the unsaturated fatty acids. As noted above, the resulting MELs are usually in the form of a mixture of compounds differing in the fatty acid residue portion of substituents R₁.

In a preferred example, the antiviral agent of the present invention contains MELs having a structure represented by formula (2) or formula (3). In formulas (2) and (3), substituents R₁ may be identical or different and are an aliphatic acyl group having 4 to 24 carbon atoms, and preferably 8 to 14 carbon atoms. Substituents R₂ may be identical or different and are a hydrogen atom or an acetyl group. Substituent R₃ is a hydrogen atom or an aliphatic acyl group having 2 to 24 carbon atoms.

Further, substituents R₁ in formulas (2) and (3) may be either a saturated aliphatic acyl group or an unsaturated aliphatic acyl group and are not particularly limited. If substituents R₁ have unsaturated bonds, the unsaturated bonds may be, for example, multiple double bonds. The carbon chain may be linear or branched. If substituents R₁ are an oxygen atomcontaining hydrocarbon group, the number and position of contained oxygen atoms are not particularly limited.

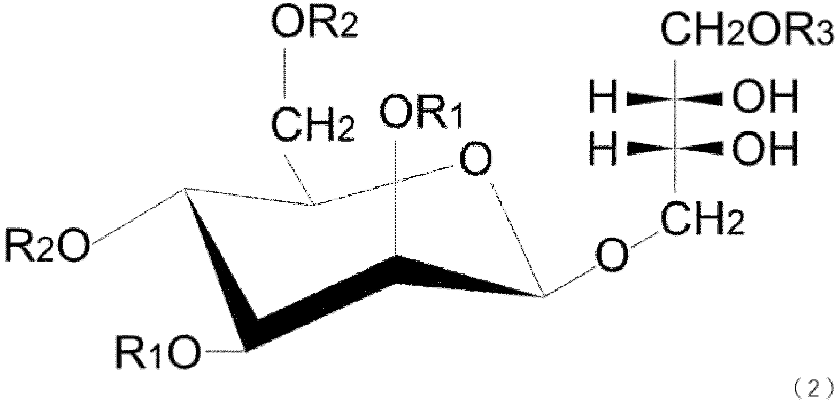

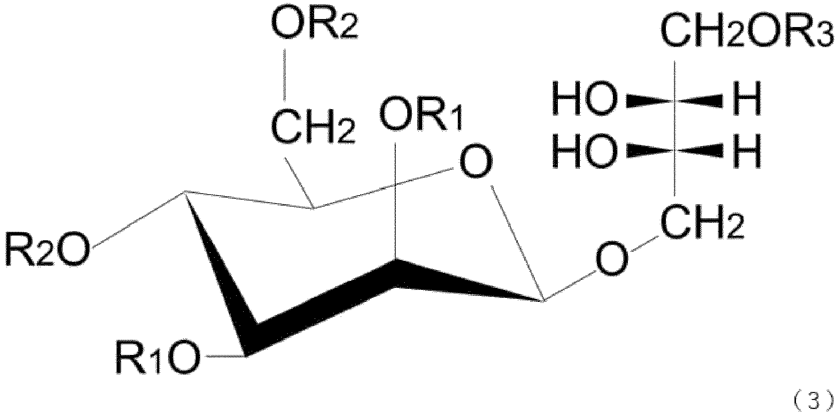

The fatty acid introduced into the erythritol moiety of an MEL may be any monovalent carboxylic acid with a long-chain hydrocarbon. The fatty acid may be a saturated fatty acid or an unsaturated fatty acid. The unsaturated fatty acid may have a plurality of double bonds. The carbon chain may be linear or branched. Further, a fatty acid derivative, which is a derivative of a fatty acid, may be used in the present invention, or a mixture of a fatty acid and a fatty acid derivative may be used in the present invention. The fatty acid or fatty acid derivative introduced into the erythritol moiety of an MEL is preferably derived from an oil, a higher fatty acid, or a synthetic ester.

The MEL preferably used in the present invention is more preferably MEL-B having the structure represented by formula (4) or (5), and still more preferably MEL-B having the structure represented by formula (4).

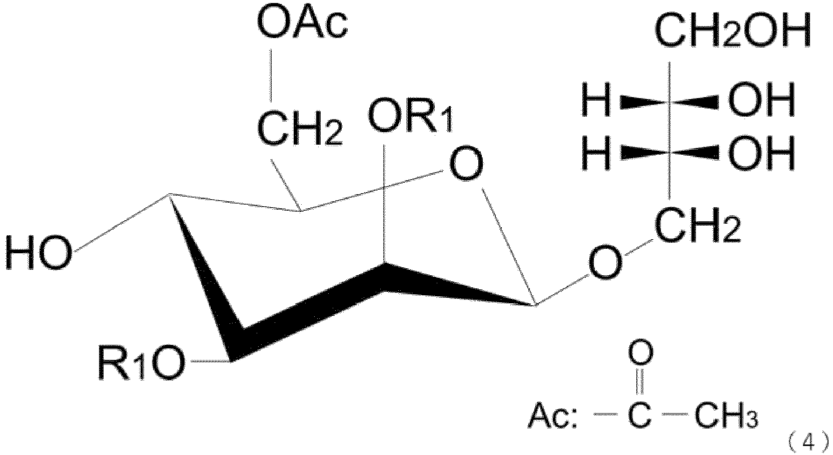

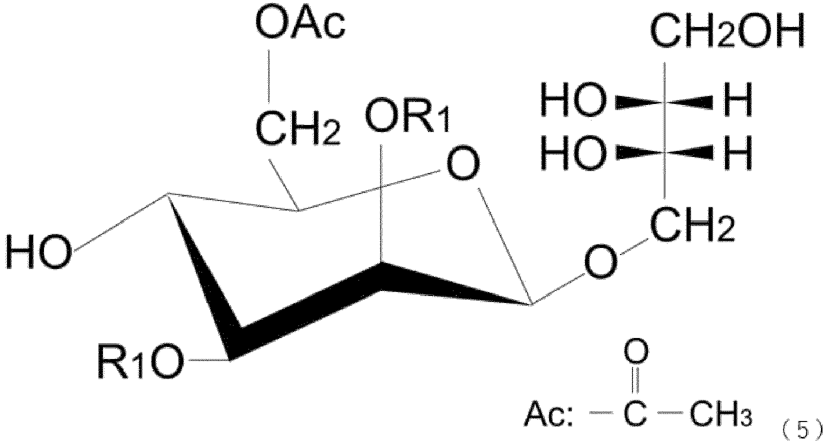

In formulas (4) and (5), substituents R₁ may be identical or different and are an aliphatic acyl group having 4 to 24 carbon atoms.

In the present invention, one kind of MELs may be used alone, or two or more kinds of MELs may be used in combination.

The target virus to which the antiviral agent of the present invention is applied is not particularly limited. The antiviral agent can be applied to both enveloped viruses (viruses with an envelope) and non-enveloped viruses (viruses with no envelope). Examples of enveloped viruses include influenza viruses (e.g., influenza virus A, and influenza virus B), rubella virus, Ebola virus, coronaviruses, measles virus, varicella zoster virus, herpes simplex viruses, mumps virus, arbovirus, RS virus, SARS virus, hepatitis viruses (e.g., hepatitis B virus and hepatitis C virus), yellow fever virus, AIDS virus, rabies virus, hantavirus, dengue virus, Nipa virus, and lyssaviruses. Examples of non-enveloped viruses include adenoviruses, noroviruses, rotaviruses, human papillomaviruses, polioviruses, enteroviruses, coxsackieviruses, human parvoviruses, encephalomyocarditis virus, polioviruses, and rhinoviruses. The target is preferably an enveloped virus, and particularly preferably an influenza virus (e.g., influenza virus A, and influenza virus B) or a coronavirus.

The antiviral agent of the present invention can be widely used in various fields requiring antiviral properties. For example, the antiviral agent can be used in various fields such as industrial, cleaning, medical, food, and daily necessities areas. The antiviral agent of the present invention can be applied to both a living organism and an article.

In the present invention, examples of living organisms include tissues and cells derived from an organism. Examples of applications of the antiviral agent to living organisms include cosmetic products, disinfectants, and cleaners. The target of application in these cases is not particularly limited, and preferable examples include various mammals, such as humans, monkeys, mice, rats, dogs, cats, rabbits, pigs, horses, cows, sheep, goats, and deer.

When applied to a living organism, the antiviral agent of the present invention can exert an antiviral effect and/or a virus-inactivating effect at a site with which the active ingredient comes into contact.

The form of the antiviral agent of the present invention is not particularly limited and may be a form commonly used in each application according to the use of the antiviral agent of the present invention.

The antiviral agent used as a cosmetic product may be in the form of, for example, liquid, gel, cream, ointment, or stick.

The antiviral agent used as a disinfectant or a cleaner may be in any form, such as liquid (solution, emulsion, suspension, etc.), semi-solid (gel, cream, paste, etc.), or solid (tablets, particulates, capsules, films, kneaded materials, molten solids, waxy solids, elastic solids, etc.). For example, the antiviral agent used in the oral cavity, more specifically, can be in the form of, for example, a dentifrice (toothpaste, liquid dentifrices, semi-liquid dentifrices, powder dentifrices, etc.), a mouthwash, a liniment, a patch, a mouth freshener, or a food (e.g., chewing gum, tablet confections, candies, gummy candies, films, troches, etc.). The antiviral agent applied to the nasal cavity, more specifically, can be in the form of, for example, a spray-type nasal drop. The antiviral agent applied to the skin can be in the form of, for example, soap, body soap, shampoo, a hair conditioner, or a spray agent.

The antiviral agent of the present invention may further optionally contain other components. Other components can be any component that can be added to, for example, cosmetic products, disinfectants, or cleaners. Examples include carriers, such as oil-based substrates, aqueous substrates, powder substrates, polymer substrates, alumina, and silica; solvents, such as water and alcohol; dispersants, such as sodium polyacrylate; emulsifiers, such as glycerin fatty acid esters and lecithin; buffers, such as citrates; stabilizers, such as sodium sulfite; excipients, such as mannitol; binders, such as crystalline cellulose; disintegrators, such as carmellose calcium; lubricants, such as magnesium stearate and talc; thickeners, such as gum arabic and xanthan gum; humectants, such as glycerin; chelating agents, such as EDTA; colorants; and flavors.

The content of MELs in the antiviral agent of the present invention depends on the kind of the active ingredient, the use, the mode of use, the application target, the state of the application target, and the like; and may be, but not limited to, for example, 0.000001 to 100 wt%, preferably 0.000001 to 80 wt%, more preferably 0.00001 to 80 wt%, still more preferably 0.0001 to 50 wt%, and particularly preferably 0.0001 to 10 wt%.

Examples of applications of the antiviral agent in articles include disinfectants and cleaners. In this case, the target of application is not particularly limited, and examples include industrial products used in various fields and raw materials of such products.

In the present invention, the term "article" means any object used in daily life other than living organisms. Specific examples include, but are not limited to, office automation equipment, household appliances, air conditioners, vacuum cleaners, desks, chairs, sofas, benches, windows, straps, handles, sheets, automatic ticket gates, automatic ticket machines, vending machines, doors, fences, handrails, tableware, cooking utensils, packaging films, packaging bags, jars, bottles, packaging materials, sinks, toilets, stationery, books, shelves, toothbrushes, glasses, air-conditioning filters, masks, clothing, such as coats, jackets, trousers, skirts, dress shirts, knitted shirts, blouses, sweaters, cardigans, nightwear, undershirts, underwear, diapers, supporters, socks, tights, pantyhose, hats, scarves, mufflers, neck gaiters, stoles, gloves, lining cloth, interlining cloth, padding materials, work garment, uniforms, and uniforms for school children, curtains, screen doors, futon materials, futon padding materials, futon covers, pillowcases, sheets, mats, carpets, towels, handkerchiefs, wall cloth, adhesive plasters, and bandages.

When applied to an article, the antiviral agent of the present invention can exert an antiviral effect and/or a virus-inactivating effect particularly at a site with which the active ingredient comes into contact.

The dosage form of the antiviral agent of the present invention is not particularly limited and can be appropriately selected according to its use. Examples of dosage forms include liquid formulations, such as liquids, emulsions, suspensions, dispersions, and aerosols; and solid or semi-solid formulations, such as wettable powder, dusts, granules, microgranules, and flowable agents.

The antiviral agent of the present invention may further optionally contain other components. Other components can be any component that can be added to, for example, cleaners or disinfectants for articles. Examples include carriers, such as oil-based substrates, aqueous substrates, powder substrates, polymer substrates, alumina, and silica; solvents, such as water and alcohol; dispersants, such as sodium polyacrylate; emulsifiers, such as glycerin fatty acid esters and lecithin; buffers, such as citrates; stabilizers, such as sodium sulfite; excipients, such as mannitol; binders, such as crystalline cellulose; disintegrators, such as carmellose calcium; lubricants, such as magnesium stearate and talc; thickeners, such as gum arabic and xanthan gum; humectants, such as glycerin; chelating agents, such as EDTA; colorants; and flavors.

The following describes the present invention in more detail with reference to Examples. However, the present invention is not limited to the examples.

### Examples

### Example 1

Virus-inactivating effects were evaluated according to the following test method using MELs suspended in purified water as a test sample.

For the sample solution for a human coronavirus inactivation test, MEL-B suspended in purified water at a concentration of 0.005% (w/v) or 0.01% (w/v) was used as a test sample.

For the sample solution for an influenza virus inactivation test, MEL-B suspended in purified water at a concentration of 0.01% (w/v) or 0.1% (w/v) was used as a test sample.

For viruses for testing, human coronavirus 229E ATCC VR-740 (human coronavirus) and influenza A virus (HINI) A/PR/8/34 ATCC VR-1469 (influenza virus) were used.

For culture of a human coronavirus, MRC-5 ATCC CCL-171 cell strain was used. The maintenance medium for use was Eagle's MEM medium Nissui (1) (manufactured by Nissui Pharmaceutical Co., Ltd.), supplemented with 2% fetal bovine serum and adjusted to a pH of around 8.

For culture of an influenza virus, MDCK (NBL-2) JCRB9029 cell line was used. The maintenance medium for use had the following composition with the pH adjusted to around 8.0.
- Eagle's MEM medium Nissui (1): 1000 mL
- 10% NaHCO₃: 14 mL
- L-glutamine (30 g/L): 9.8 mL
- 100 × MEM vitamin solution: 30 mL
- 10% albumin: 20 mL
- 0.25% trypsin: 20 mL

The virus culture broth described above was centrifuged, and 0.1 mL of the obtained supernatant was added to 1 mL of a sample solution and allowed to act for 1 minute or 15 minutes at room temperature. For the control, 0.1 mL of purified water was allowed to act on the sample solution for 0 minutes or 15 minutes.

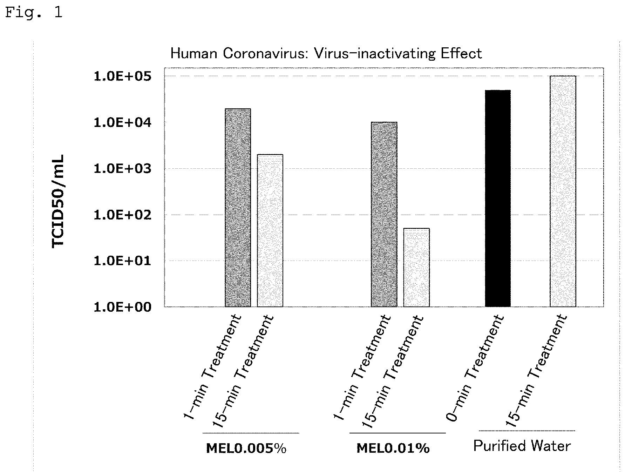

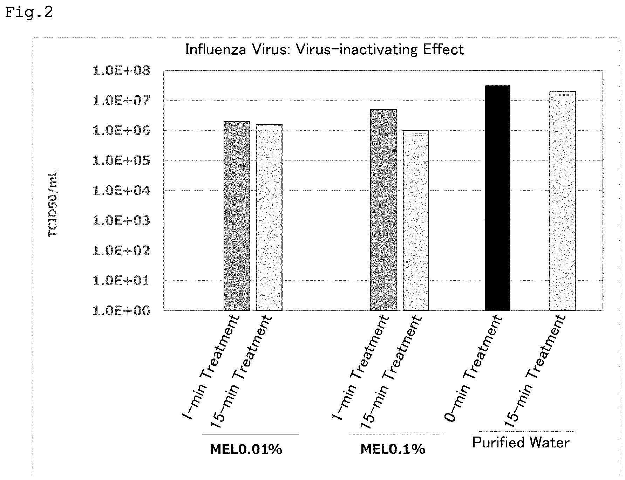

The acted-on solution was inoculated into culture cells and cultured in a maintenance medium for 7 days under 5% CO₂ conditions. After the culture, the cells was observed to examine whether there were morphological changes (cytopathic effect) in the cells. The concentration of the sample solution in which 50% of the cultured cells were infected with the virus was calculated to determine the virus-inactivating effect per milliliter (TCID50/ml). Figs. 1 and 2 show the results.

Under the condition of 0.005% MEL, the human coronavirus infectivity titer was reduced by about one order. Further, under the condition of 0.01% MEL, the viral infectivity titer was reduced by two orders of magnitude or more. Given these facts, MELs have an antiviral effect or virus-inactivating effect on a human coronavirus even at a very low concentration.

Under the conditions of 0.01% MEL and 0.1% MEL, the infectivity titer of the influenza virus was reduced by about one order. The antiviral effect or virus-inactivating effect of the present invention appears to be a result of physicochemical interaction between MELs and the viral lipid membrane due to the interfacial activity of MELs.

### Example 2

The following is an example of formulations for a disinfectant in the present invention.
- MEL-B 0.1% (w/v)
- Bis-PEG/PPG-[14-20]/[5-20]-dimethicone 1% (w/v)
- ethanol 80% (w/w)
- Purified water present in an amount to bring the entire composition to 100 mass%

### Example 3

The following is an example of formulations for a disinfectant in the present invention.
- MEL-B 0.1% (w/v)
- Ethanol 35% (w/v)
- Purified water present in an amount to bring the entire composition to 100 mass%

### Example 4

The following is an example of formulations for a skin lotion in the present invention (w/v%).
- Polyoxyethylene (60EO) hydrogenated castor oil 0.03%
- Polyethylene glycol 4000 1%
- Ethanol 8%
- 1,3-Butylene glycol 7%
- Glycerin 5%
- Carboxyvinyl polymer 0.02%
- Acrylic acid-alkyl methacrylate copolymer 0.16%
- Olive oil 0.4%
- Methylpolysiloxane 0.4%
- Potassium hydroxide 0.065%
- Fragrance 0.1%
- Dipotassium glycyrrhizinate 0.1%
- Sodium carboxymethyl-β-glucan 0.1%
- MEL-B 0.01%
- Purified water present in an amount to bring the entire composition to 100 mass%

### Example 5

The following is an example of formulations for an emulsion in the present invention (w/v%).
- Stearic acid 0.24%
- Sorbitan monostearate 0.5%
- Cetanol 0.5%
- POE (45)-stearate 0.7%
- Cetyl palmitate 0.25%
- Petrolatum 3.75%
- Liquid paraffin 0.9%
- Solid paraffin 1.6%
- PEG4000 2.25%
- 1,3-Butylene glycol 3%
- Methyl parahydroxybenzoate 0.3%
- Glycerin 3%
- Xanthan gum 0.03%
- Alkyl-modified carboxyvinyl polymer 0.15%
- Triethanolamine 0.15%
- MEL-B 0.1%
- Sucrose laurate 0.05%
- 1,3-Butanediol 10%
- Purified water present in an amount to bring the entire composition to 100 mass%

### Example 6

The following is an example of formulations for a cleaner for articles in the present invention (wt%).
- Sodium hydroxide 3%
- Sodium methacrylate 5%
- Propylene glycol monomethyl ether 4%
- Sodium gluconate 0.5%
- Tetrasodium ethylenediaminetetraacetate 0.5%
- Sodium decanoate 0.7%
- Sodium 2-ethylhexanoate 2%
- MEL-B 1%
- Tocopherol acetate 0.01%
- Sodium sulfite 2%
- Xanthan gum 0.3%

### Example 7

The following is an example of formulations for a cleaner for animals in the present invention (w/v%).
- Sodium benzoate 0.4%
- Edetate-2-sodium 0.05%
- 1,3-Butylene glycol 2%
- O-[2-hydroxy-3-(trimethylammonio)propyl]hydroxyethylcellulose chloride 0.5%
- Cocamidopropyl propyl betaine liquid 30%
- Sodium cocoyl alanine solution 30%
- Coconut oil fatty acid monoethanolamide 2.5%
- Glycerin 2.5%
- Phenoxyethanol 0.25%
- MEL-B 2.0%
- Silicone emulsion (dimethiconol) 3%
- Purified water present in an amount to bring the entire composition to 100 mass%

### Industrial Applicability

Using a composition containing MELs, the present invention has enabled the provision of an antiviral agent that is suitable not only for application in articles but also for application in living organisms, and that is safer for living organisms. A preventive effect against viral infection is expected in a wide range of areas such as industry, cleaning, medical, food, and daily necessities.

## Claims

1. An antiviral agent comprising a mannosylerythritol lipid (MEL) as an active ingredient.

2. The antiviral agent according to claim 1, wherein the content of the mannosylerythritol lipid (MEL) is 0.000001 to 100 wt%.

3. The antiviral agent according to claim 2, wherein the content of the mannosylerythritol lipid (MEL) is 0.000001 to 80 wt%.

4. The antiviral agent according to claim 2, wherein the content of the mannosylerythritol lipid (MEL) is 0.0001 to 10 wt%.

5. The antiviral agent according to any one of claims 1 to 4, wherein the mannosylerythritol lipid (MEL) is any one selected from the group consisting of MEL-A, MEL-B, MEL-C, and MEL-D.

6. The antiviral agent according to any one of claims 1 to 5, wherein the mannosylerythritol lipid (MEL) has a structure of formula (2): 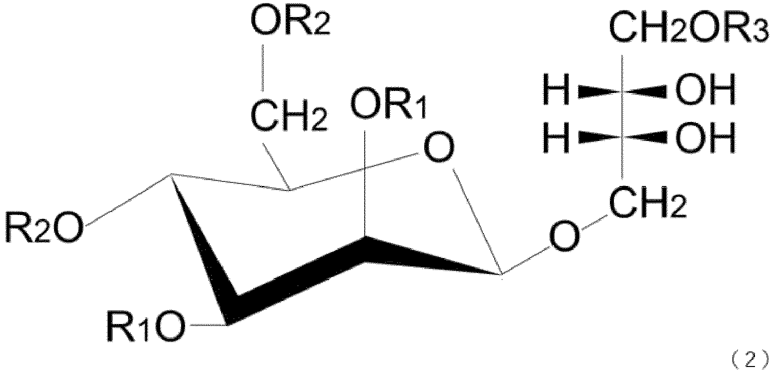 wherein R₁s are an aliphatic acyl group having 4 to 24 carbon atoms and may be identical to or different from each other, R₂s are a hydrogen atom or an acetyl group and may be identical to or different from each other, and R₃ is a hydrogen atom or an aliphatic acyl group having 2 to 24 carbon atoms.

7. The antiviral agent according to any one of claims 1 to 5, wherein the mannosylerythritol lipid (MEL) has a structure of formula (3): 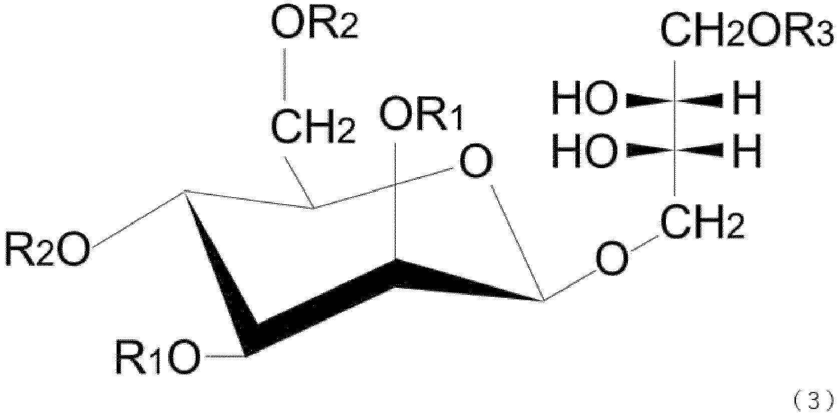 wherein R₁s are an aliphatic acyl group having 4 to 24 carbon atoms and may be identical to or different from each other, R₂s are a hydrogen atom or an acetyl group and may be identical to or different from each other, and R₃ is a hydrogen atom or an aliphatic acyl group having 2 to 24 carbon atoms.

8. The antiviral agent according to any one of claims 1 to 5, wherein the mannosylerythritol lipid (MEL) is MEL-B.

9. The antiviral agent according to claim 8, wherein the MEL-B has a structure of formula (4): 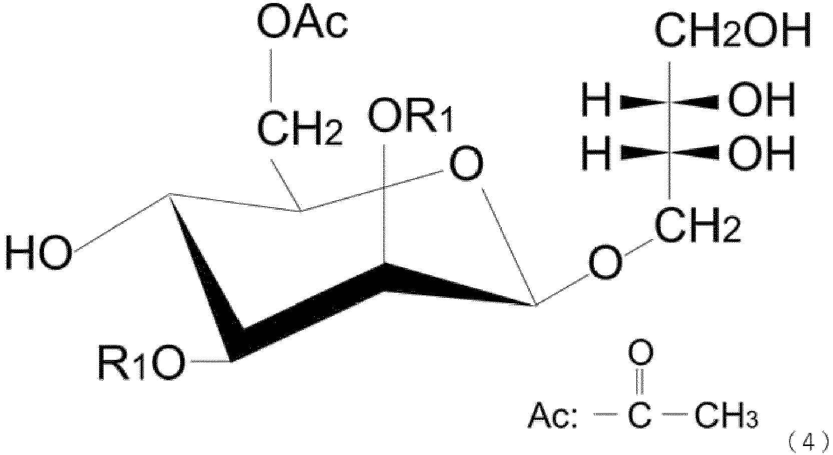 wherein R₁s are a saturated or unsaturated, linear or branched aliphatic acyl group having 2 to 20 carbon atoms and may be identical to or different from each other.

10. The antiviral agent according to claim 8, wherein the MEL-B has a structure of formula (5): 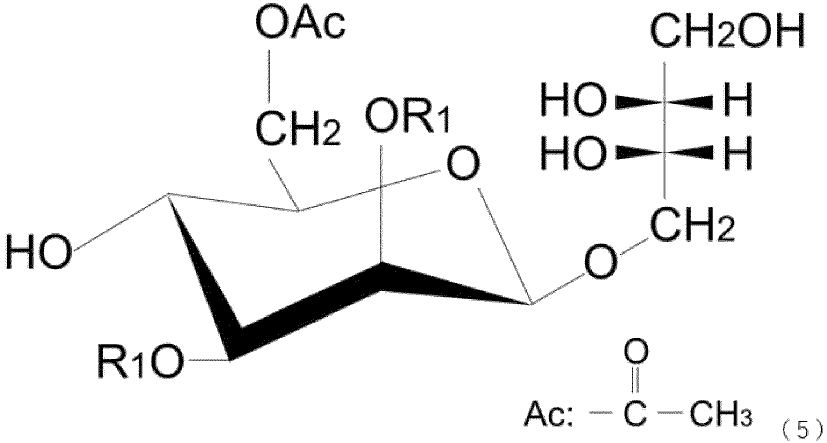 wherein R₁s are a saturated or unsaturated, linear or branched aliphatic acyl group having 2 to 20 carbon atoms and may be identical to or different from each other.

11. The antiviral agent according to any one of claims 1 to 10, wherein a target virus is an enveloped virus.

12. The antiviral agent according to claim 11, wherein the target virus is an influenza virus.

13. The antiviral agent according to claim 11, wherein the target virus is a human coronavirus.

14. The antiviral agent according to any one of claims 1 to 13, which is for use in a living organism.

15. The antiviral agent according to any one of claims 1 to 13, which is for use in an article.

16. A cosmetic product comprising the antiviral agent of any one of claims 1 to 14.

17. A disinfectant comprising the antiviral agent of any one of claims 1 to 15.

18. A cleaner comprising the antiviral agent of any one of claims 1 to 15.
